(19) Europäisches Patentamt
European Patent Office
Office européen des brevets

(11) **EP 3 851 540 A1**

(12) **EUROPÄISCHE PATENTANMELDUNG**

(43) Veröffentlichungstag:
21.07.2021   Patentblatt 2021/29

(51) Int Cl.:
*C12Q 1/6806* $^{(2018.01)}$    *C12Q 1/6886* $^{(2018.01)}$

(21) Anmeldenummer: 20152341.2

(22) Anmeldetag: 17.01.2020

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
**KH MA MD TN**

(71) Anmelder: **SensID GmbH**
**18057 Rostock (DE)**

(72) Erfinder:
• **STROH, Eileen**
**18311 Ribnitz (DE)**
• **BOLLMANN, Andreas**
**12685 Berlin (DE)**
• **NOWACK, Björn**
**18119 Seebad-Warnemünde (DE)**

(74) Vertreter: **Simandi, Claus**
**Badehausallee 55**
**27476 Cuxhaven (DE)**

(54) **NEUES VERFAHREN ZUR BESTIMMUNG DER ALLELFREQUENZ / MUTATIONSRATE UND DIAGNOSTIK**

(57)    Die vorliegende Erfindung betrifft ein neues Verfahren zur Bestimmung der Allelfrequenz und / oder Mutationsrate in Nukleinsäuren, insbesondere in Tumor-Nukleinsäuren im Rahmen einer iwS. Polymerase-Chain-Reaction (PCR) sowie eine Diagnostik hierzu, wobei mindestens eine Referenznukleinsäure (RN) und eine Mutationssequenz zur Referenznukleinsäure eingesetzt wird. Diese Referenznukleinsäure und Mutationssequenz erlaubt die Validierung von Verfahren zur Polymerase-Chain-Reaction (PCR), insbesondere in Abhängigkeit von Geräteparametern und Probenvorbereitung. Weiterhin umfasst die Erfindung ein zugehöriges Diagnose- und Prognoseverfahren, insbesondere zur Tumordiagnose im Zuge einer Liquid Biopsy.

Kalibrierungskurve

Fig. 3

**Beschreibung**

Die vorliegende Erfindung betrifft ein neues Verfahren zur Bestimmung der Allelfrequenz und / oder Mutationsrate in Nukleinsäuren, insbesondere in Tumor-Nukleinsäuren im Rahmen einer iwS. Polymerase-Chain-Reaction (PCR) sowie eine Diagnostik hierzu, wobei mindestens eine Referenznukleinsäure (RN) und eine Mutationssequenz zur Referenznukleinsäure eingesetzt wird. Diese Referenznukleinsäure und Mutationssequenz erlaubt die Validierung von Verfahren zur Polymerase-Chain-Reaction (PCR), insbesondere in Abhängigkeit von Geräteparametern und Probenvorbereitung. Weiterhin umfasst die Erfindung ein zugehöriges Diagnose- und Prognoseverfahren, insbesondere zur Tumordiagnose im Zuge einer Liquid Biopsy.

Gegenwärtige Verfahren zum Nachweis von Tumoren wie klinischchemische Untersuchungen in Körperflüssigkeiten und Gewebeproben oder bildgebende Verfahren führen trotz aller Fortschritte in den letzten Jahren zu einem meist zu späten Nachweis von malignen Veränderungen. So ist die Hauptursache der hohen Mortalitätsrate von Tumorpatienten nicht das Auftreten von Primärtumoren, sondern von Metastasen. Um das Auftreten von Metastasen zu verhindern, ist ein so früh wie möglicher Nachweis von malignen Veränderungen erforderlich. Ferner bedarf es Verfahren, um maligne Zellen von normalen Zellen richtig unterscheiden zu können. Sowohl falsch-positive als auch falsch-negative Befunde haben fatale Konsequenzen für die Betroffenen. Ebenso sind Verfahren erforderlich, die eine richtige Prognose von Tumorpatienten gestatten und eine entsprechend auf den Patienten individuell abgestimmte Therapie erlauben.

Bisher werden in Körperflüssigkeiten wie Blut-, Urin-, Sputumoder Gewebeproben sogenannte Tumormarker, insbesondere Onkogene bestimmt. Hierbei handelt es sich um Bestandteile von Tumorzellen, die verstärkt oder vermindert gebildet werden und deren Veränderungen in Körperflüssigkeiten wie Blut, Plasma oder Serum nachgewiesen werden können. Diese Tumormarker erlauben jedoch kein generelles Screening auf Tumorerkrankungen, da ihre diagnostischen Spezifitäten und Sensitivitäten meistens zu gering sind. Wenngleich diese Tumormarker gegenüber bildgebenden Verfahren oft frühzeitiger maligne Veränderungen anzeigen könnten, gestatten diese bisher keinen hinreichenden Nachweis von malignen Veränderungen.

Die Isolierung, Charakterisierung und Analyse intrazellulärer Bestandteile, vor allem von Nukleinsäuren (Ribonukleinsäuren, RNA und Desoxyribonukleinsäuren, DNA), ist von großer Bedeutung für die moderne Molekularbiologie. Spätestens seit der Erfindung der Polymerasekettenreaktion (PCR) im Jahre 1983 ist eine Vielzahl von Nukleinsäurediagnostik-Verfahren entwickelt worden, die beispielsweise zum Nachweis von Krankheiten und Krankheitserregern genutzt werden.

Im Stand der Technik wird solches Zellmaterial in den nachfolgenden Schritten, wie Probenaufbereitung, Extraktion, Konzentration, Isolierung, Reinigung, reverse Transkription, Amplifikation und Detektion für die Diagnostik bereitgestellt.

Aufgrund der leistungsfähigen Amplifikation von Tumor-DNA mittels PCR, insbesondere die "qPCR" (real-time quantitative Polymerase Chain Reaction), "digital droplet PCR" (ddPCR), oder "next generation sequencing" (NGS, bzw.

Parallelsequenzierung wie "Massive Parallel Sequencing") ist in den letzten Jahren die so genannte nicht-invasive Liquid Biopsy in der Tumordiagnostik etabliert worden, wobei zirkulierende freie DNA in einer Probe untersucht wird. Solche zirkulierende freie DNA (kurz: cfDNA) kann beispielsweise aus einer Krebs-/Tumorzelle stammen (so genannte: ctDNA).

In Krebspatienten liegt die durchschnittliche cfDNA Menge höher als in gesunden Probanden, insbesondere ist das cfDNA Plasma Level bei Krebspatienten im fortgeschrittenen Stadium höher als im milden/frühen Stadium. ctDNA macht z.B. 1% der cfDNA aus (0,01-90% der normalen cfDNA, je nach Stadium und Lokalisation des Tumors).

Es besteht jedoch ein hohes Bedürfnis an einem Standard, so dass nicht nur relative, sondern auch absolute diagnostische Aussagen getroffen werden können. Der Einsatz eines solchen Standards wird zum Beispiel in WO2018094183A1 beschrieben. Ebenfalls wird eine zugehörige Diagnostik offenbart (Anspruch 183).

Derzeit stehen für Mutationen, die es für die Tumordiagnostik nachzuweisen gilt, sowohl kommerzielle Tests zur Verfügung oder es werden Laboreigene Tests (lab-developed test, LDT) angewendet. Als Qualitätskontrollen für die Validierung bzw. Kalibrierung werden dabei derzeit als Stand der Technik Laborinterne Standards wie z.B. Plasmid-DNA oder synthetische Oligonukleotide u.v.a. eingesetzt.

Nachteilig ist jedoch, dass diese Labor-internen Standards zwecks Validierung oder Kalibrierung eines Gerätes oder eines Verfahrens selbst verdünnt werden müssen und hierfür kein automatisierter Prozess zur Verfügung steht, sodass eine Standardisierung zumeist fehlerbehaftet ist. Ferner ist die Stabilität solcher Verdünnungen nicht gewährleistet und je nach Quelle wie z.B. Plasmid-DNA aus E. coli besteht die Gefahr einer RNA-Kontamination. Folglich kann die mittels PCR ermittelte Kopienzahl mangels hinreichender Standardisierung relativ und absolut falsch sein.

Daher gilt es, die Standardisierung anhand einer humanen Referenznukleinsäure weiter zu verbessern, wobei eine weitere Mutationssequenz vorliegt. Die humane Referenznukleinsäure als auch Mutationssequenz bilden einen Standard.

Überraschenderweise konnten die Erfinder feststellen, dass zu einem solchen Standard die Verwendung einer humanen Serumoder Plasmaprobe, welche jedoch frei von DNA ist, eine verbesserte Validierung als auch Diagnose erlaubt.

Die Aufgabe der vorliegenden Erfindung ist daher die Bereitstellung eines verbesserten Verfahrens zum Nachweis einer Allelfrequenz und / oder Mutationsrate in einer Probennukleinsäure samt zugehöriger Diagnose, wobei ein geeigneter Standard eingesetzt werden soll.

Die Aufgabe wird gelöst durch ein Verfahren zur Validierung eines Polymerase-Chain-Reaction (PCR)-Verfahrens mittels Bestimmung der Allelfrequenz und / oder Mutationsrate in Nukleinsäuren mit den Schritten:

a.) Bereitstellen mindestens einer humanen Referenznukleinsäure (=Wildtyp) und einer humanen Nukleinsäure aufweisend eine oder mehrere Mutationen (=Mutationsnukleinsäure) zur Referenznukleinsäure in einer DNA-freien Serum- oder Plasmaprobe, wobei eine bestimmte Allelfrequenz und / oder Mutationsrate vorgegeben ist, und optional
b.) Bereitstellen einer humanen Referenznukleinsäure (=Wildtyp) aus a.) in einer DNA-freien Serum- oder Plasmaprobe, wobei die Allelfrequenz und / oder Mutationsrate 0 % ist,
und optional
c.) Bereitstellen einer DNA-freien Serum- oder Plasmaprobe,

wobei die bestimmte Allelfrequenz in a.) nachgewiesen wird, und ggfs. mit b.) oder / und c.) verglichen wird.

Das vorstehende Verfahren wird nachstehend "erfindungsgemäßes Validierungsverfahren" genannt.

Eine vorgenannte "bestimmte Allelfrequenz" ist gleich oder größer als 0% und kann insbesondere Werte annehmen, wie 0,01 %, 0,1%, 0,5 %, 1 %, 2,5, 5% u.v.a.

Im Sinne dieser Erfindung bedeutet "Referenznukleinsäure" eine beliebige und bekannte humane Wildtypsequenz eines Allels. Solche Allele können Datenbanken, wie
COSMIC: https://cancer.sanger.ac.uk/cosmic,
Targeted Cancer Care:
http://targetedcancercare.massgeneral.org/My-Trial-Guide/Diseases/Lung-Cancer/KRAS/G12C-(c-34G-T).aspx, oder
OncoKB: https://oncokb.org/, My Cancer Genome:
https://www.mycancergenome.org/ entnommen werden.

In einer bevorzugten Ausführungsform beträgt der Gehalt an einer Referenznukleinsäure, vorzugsweise 20 - 600 ng, insbesondere 400 ng DNA in Serum oder Plasma, wie z.B. 400 ng/5ml, entspricht 80ng/ml, entspricht 0,08 ng/$\mu$l DNA in Serum oder Plasma.

Weiterhin ist bevorzugt, dass die Referenznukleinsäure eine Größe von 50 bp bis 500 bp aufweist.

Im Sinne dieser Erfindung bedeutet "humane Nukleinsäure aufweisend eine oder mehrere Mutationen" bzw. "Mutationssequenz oder Mutationsnukleinsäure" eine solche Sequenz die gegenüber einer humanen Wildtypsequenz eines Allels als Referenznukleinsäure ein oder mehrere Mutationen aufweist. Hierbei können gegenüber der humanen Wildtypsequenz eines Allels bekannte Mutationssequenzen eingesetzt oder künstliche Mutationen eingeführt werden. Methoden zur Herstellung solcher künstlichen Mutationen sind im Stand der Technik beschrieben. Wesentlich ist, dass im erfindungsgemäßen Verfahren diese Referenzsequenz als auch Mutationssequenz bekannt sind (z.B. beschrieben anhand der Nukleinsäuresequenz). Weiterhin ist bevorzugt, dass die Mutationssequenz mindestens einen Tumormarker, z.B. Onkogen, aufweist mit bekannten Mutationen. Ferner können solche Tumormarkersequenzen oder Onkogene weitere künstliche Mutationen aufweisen.

In einer bevorzugten Ausführungsform ist der Gehalt an einer Mutationssequenz weniger als der Gehalt an Referenzsequenz. Weiterhin ist bevorzugt, dass die Mutationssequenz eine Größe von 50 bp bis 500 bp aufweist.

Weiterhin ist bevorzugt, dass die Referenznukleinsäure und Mutationssequenz jeweils in einer bestimmten Konzentration vorliegen.

Allelfrequenz oder Mutationsrate bedeutet erfindungsgemäß das Verhältnis von Referenznukleinsäure zur Mutationssequenz (RN/MS), beispielsweise über die Anzahl der vorliegenden Kopien von Referenznukleinsäure zur Mutationssequenz oder das Verhältnis anhand der Konzentration von Referenznukleinsäure zur Mutationssequenz.

Beispielsweise beträgt die Allelfrequenz oder Mutationsrate 4,76 %, falls 200 Mutationssequenzen ("mutiertes Allel") zu 4.000 Kopien einer Referenznukleinsäure ("wildtyp Allel") (Hier: 4,76 % = (200 x 100 %) / (200 + 4000)) gemeinsam vorliegen. Im Sinne der Allelfrequenz oder Allelhäufigkeit beträgt folglich diese 4,76 %, d.h. die Häufigkeit der Allel-Mutation bzw. Mutationssequenz beträgt 4,76 % bzw. die Mutationsrate beträgt 4,76 %.

Auf diese Weise können erfindungsgemäße Validierungsproben bereitgestellt werden, die eine bestimmte bzw. eingestellte Allelfrequenz (Allelhäufigkeit) oder Mutationsrate aufweisen.

Im Sinne dieser Erfindung bedeutet Validierung, dass anhand der vorgegebenen Validierungsproben die bestimmte bzw. eingestellte Allelfrequenz oder Mutationsrate im Zuge einer Polymerase-Chain-Reaction (PCR) nachgewiesen werden können. Dies kann einerseits von Geräteparametern abhängen, andererseits von der Durchführung der Polymerase-Chain-Reaction (PCR) und insbesondere von der Probenvorbereitung. Die Validierungsproben können mittels eines Kits bereitgestellt werden.

Sofern die Validierungsproben in ihrer Allelfrequenz / Mutationsrate bei der Durchführung der Polymerase-Chain-Reaction (PCR) nicht wiedergefunden werden, ist die Sensitivität nicht gegeben bzw. die Detektionsgrenze zu hoch, so dass ein Nachweis erfolgen kann. Weiterhin besteht die Möglichkeit, dass bei der Probenvorbereitung der Validierungsproben für eine PCR zu wenig DNA-Material extrahiert werden konnte. Folglich kann die Extraktionseffizienz mangelhaft sein, mit Folgewirkung für die Probenvorbereitung bei einer parallelen Patienten-, Probandenprobe einer Probennukleinsäure.

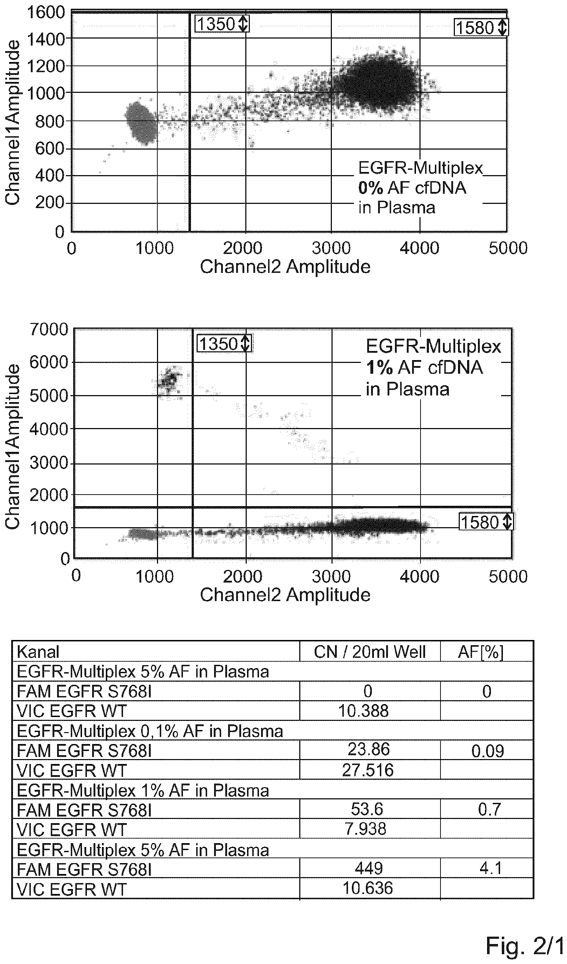

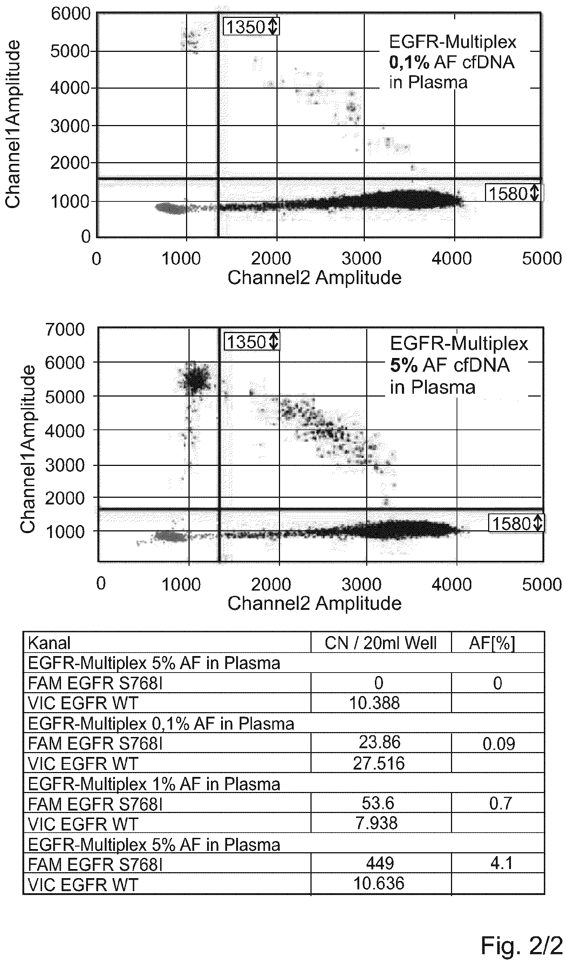

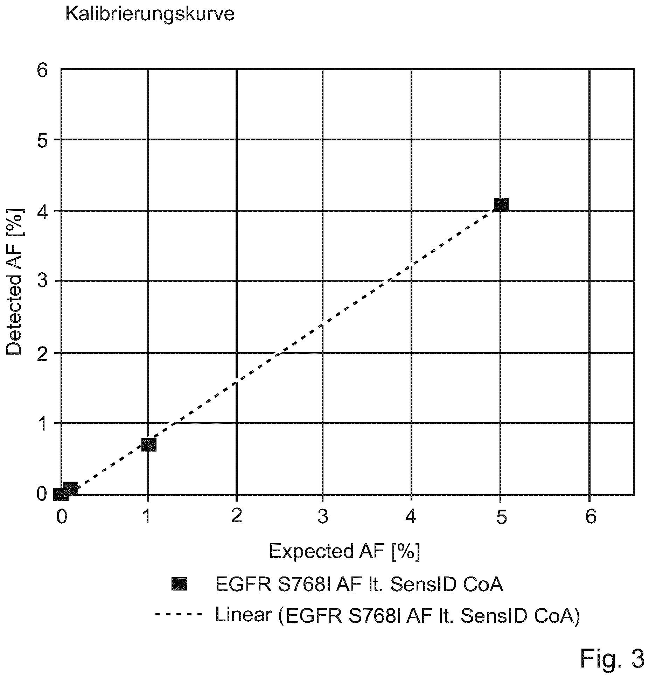

Eine beispielhafte Validierung ist in den Beispielen und in den Figuren 2 und 3 beschrieben.

In einer weiteren bevorzugten Ausführungsform können Kalibrierungs- oder Validierungskurven erstellt werden.

Eine beispielhafte Kalibrierungskurve ist in den Beispielen und Figur 3 beschrieben.

Überraschender Weise können auf diese Weise vorteilhaft geringe Mengen an Probennukleinsäure mit hinreichender Spezifität und Sensitivität erfasst werden. Dies erlaubt eine frühzeitige Aussage über die Tumoraktivität, insbesondere Wahrscheinlichkeit einer Metastasierung. Eine bevorzugte Probennukleinsäure ist cfDNA oder ctDNA eines Patienten oder Probanden.

Daher betrifft die Erfindung ein Verfahren zur Bestimmung der Allelfrequenz und / oder Mutationsrate mindestens einer Probennukleinsäure mittels eines PCR-Verfahrens, wobei das erfindungsgemäße Validierungsverfahren erfolgt ist. Besonders vorteilhaft kann anhand der Kalibrierung bzw. Validierung eine quantitative Bestimmung der Probennukleinsäure erfolgen.

Eine weitere besondere Ausführungsform der Erfindung betrifft ein Verfahren zur Diagnose oder Prognose einer Tumorerkrankung, wobei eine Änderung der Allelfrequenz und / oder Mutationsrate einer Probennukleinsäure aus einer ersten Probe und einer zweiten und / oder weiteren Probe auf die Früherkennung und Erkennung, zur Beurteilung des Schweregrades und zur therapiebegleitenden Verlaufsbeurteilung schließen lässt, wobei eine Kalibrierung mit Hilfe des erfindungsgemäßen Validierungsverfahrens erfolgt ist.

Insbesondere kann die zweite oder weitere Probe zu einem jeweiligen späteren Zeitpunkt von einem Patienten entnommen werden.

Das Bereitstellen einer DNA-freien Serum- oder Plasmaprobe erlaubt besonders vorteilhaft, die Nachweisgrenze der Standardnukleinsäure als auch der Probennukleinsäuren zu validieren. Eine besonders vorteilhafte Anwendung betrifft daher die Validierung von Geräten zur Durchführung einer Polymerase-Chain-Reaction (PCR), insbesondere next generation sequencing (NGS), wobei das erfindungsgemäße Verfahren durchgeführt wird.

Besonders vorteilhaft kann auf diese Weise eine exakt vorgegebene Menge an DNA-Material bzw. Konzentration des erfindungsgemäßen Standards in eine Probe eingebracht werden, wobei diese Probe eine Patientenserum- oder Plasmaprobe weitgehend simuliert und für die Durchführung einer Tumordiagnostik hochgradig geeignet ist.

Weiterhin ist bevorzugt, dass die Konzentration an Mutationsnukleinsäure vorzugsweise $4 \times 10E-05$ fg/$\mu$l bis 0,03 fg/$\mu$l beträgt.

Die erfindungsgemäße Mutationssequenz weist vorzugsweise einen Tumormarker auf, insbesondere ein Onkogen ausgewählt aus der Gruppe MTOR, MPL, NRAS, PARP1, AKT3, DNMT3A, MSH2, IDH1, VHL, MLH1, MYD88, CTNNB1, ATR, PIK3CA, FGFR3, PDGFRA, KIT, FBXW7, APC, GABRG2, NPM1, EGFR, MET, BRAF, EZH2, JAK2, GNAQ, RET, PTEN, ATM, KRAS, PTPNII, FLT3, RB1, PARP2, ARHGAP5, AKT1, RAD51, IDH2, TP53, NF1, SMAD4, AKT2, ERCC1, GNAS, ERBB2, FOXL2, NOTCH1, NTKR.

Tabelle 1, Onkogene (siehe z.B. "COSMIC" (supra)):

| Gene | Position | REF | ALT | Strand | CDS | AA | COSID |
|---|---|---|---|---|---|---|---|
| MTOR | 11291097 | T | A | - | 2664 A>T | L888F | COSM94356 |
| MPL | 43815009 | G | T | + | 1544 G>T | W515L | COSM18918 |
| NRAS | 115256529 | T | C | - | 182 A>G | Q61R | COSM584 |
| PARPI | 226551691 | TC | T | - | 2738 delG | G913fs*4 | COSM21691 |
| AKT3 | 243809253 | T | A | - | 371 A>T | Q124L | COSM48227 |
| DNMT3A | 25457243 | G | A | - | 2644 C>T | R882C | COSM53042 |
| MSH2 | 47705449 | TG | T | + | 2250 delG | G751fs*12 | COSM111644 |
| MSH2 | 47705558 | ACT | A | + | 2359 2360 delCT | L787fs* 11 | COSM26122 |
| IDHI | 209113113 | G | A | - | 394C>T | R132C | COSM28747 |
| VHL | 10188282 | TTGAC | T | + | 426429 del TGAC | G144fs*14 | COSM18578 |
| MLHI | 37067240 | T | A | + | 1151 T>A | V384D | COSM26085 |
| MVD88 | 38182641 | T | c | + | 794 T>C | L265P | COSM85940 |
| CTNNBI | 41266124 | A | G | + | 121 A>G | T41A | COSM5664 |
| ATR | 142254972 | GCTITIAT | G | - | 3790 3796 del ATAAAAG | 11264fs*24 | COSM20627 |
| PIK3CA | 179218303 | G | A | + | 1633 G>A | E545K | COSM763 |
| PIK3CA | 179218304 | A | C | | 1634A>C | E545A | COSM12458 |
| PIK3CA | 179218305 | G | T | | 1635G>T | E545D | COSM765 |
| PIK3CA | 179218304 | A | G | | 1634A>G | E545G | COSM764 |
| PIK3CA | 179218294 | G | A | | 1624G>A | E542K | COSM760 |
| PIK3C A | 179210192 | T | C | | 1258T>C | C420R | COSM757 |
| PIK3C A | 179218306 | C | G | | 1636C>G | Q546E | COSM6147 |
| PIK3C A | 179218307 | A | G | | 1637A>G | Q546R | COSM12459 |
| PIK3C A | 179234297 | A | G | + | 3140 A>G | H1047R | COSM775 |
| PIK3C A | 179234297 | A | T | | 3140A>T | H1047L | COSM776 |

| Gene | Position | REF | ALT | Strand | CDS | AA | COSID |
|------|----------|-----|-----|--------|-----|-----|-------|
| PIK3C A | 179234296 | C | T | | 3139C>T | H1047Y | COSM774 |
| PIK3C A | 178952149 | c | CA | + | 3204 3205 ins A | N1068fs*4 | COSM12464 |
| FGFR 3 | 1803568 | c | G | + | 746 C>G | S249C | COSM715 |
| PDGF RA | 55141048 | T | TA | + | 16941695 ins A | S566fs*6 | COSM28053 |
| PDGF RA | 55152093 | A | T | + | 2525 A>T | D842V | COSM736 |
| KIT | 55599321 | A | T | + | 2447 A>T | D816V | COSM1314 |
| FBXW 7 | 153249384 | c | T | | 1394 G>A | R465H | COSM22965 |
| APC | 112175538 | GC | G | + | 4248 del C | 11417fs*2 | COSM18584 |
| APC | 112175639 | c | T | + | 4348 C>T | R1450* | COSM13127 |
| APC | 112175957 | A | AA | + | 46664667 ins A | T1556fs*3 | COSM18561 |
| GABR A6 | 161117296 | G | c | + | 763 G>C | V255L | COSM70853 |
| GABR G2 | 161580301 | A | G | + | 1355 A>G | Y452C | COSM74722 |
| NPM | 170837547 | G | GTCTG | + | 863864 ins TCTG | W288fs*12 | COSM17559 |
| EGFR | 55174787 | GGAAT IAAG AGAAG CA | G | + | 2236 2250 del 15 | E746 A750 del ELREA | COSM6225 |
| EGFR | 55249012 | c | CGGT | + | 23102311 ins GGT | 0770 N771 ins G | COSM12378 |
| EGFR | 55181378 | c | T | + | 2369 C>T | T790M | COSM6240 |
| EGFR | 55191822 | T | G | + | 2573 T>G | L858R | COSM6224 |
| EGFR | 55174014 | G | A | | 2155G>A | G719S | COSM6252 |
| EGFR | 55191831 | T | A | | 2582T>A | L861Q | COSM6213 |
| EGFR | 55181312..5 5181313 | | insTGTGGCCAG | | 2303_2304insTG TGGCCAG | V769_D770ins ASV | COSM20884 |
| EGFR | 55174791..5 5174814 | | delTCTCCGAAAGCCAA CAAGGAAATC | | 2254_2277del24 | S752 1759delS PKANKEI | COSM6256 |

| Gene | Position | REF | ALT | Strand | CDS | AA | COSID |
|------|----------|-----|-----|--------|-----|-----|-------|
| EGFR | 55181312 | G | T | | c.2303G>T | S768I | COSM6241 |
| MET | 116423428 | T | G | + | 3757 T>G | Y1253D | COSM700 |
| BRAF | 140753336 | T | A | - | 1799 T>A | V600E | COSM476 |
| EZH2 | 148508727 | T | A | - | 1937 A>T | V646F | COSM37028 |
| JAK2 | 5073770 | G | T | + | 1849 G>T | V617F | COSM12600 |
| GNAQ | 80409488 | T | G | - | 626 A>C | Q209P | COSM28758 |
| RET | 43617416 | T | c | + | 2753 T>C | M918T | COSM965 |
| PTEN | 89692904 | c | T | + | 388 C>T | R130* | COSM5152 |
| PTEN | 89717716 | A | AA | + | 741 742 ins A | P248fs*5 | COSM4986 |
| PTEN | 89717774 | AA | A | + | 800 del A | K267fs*9 | COSM5809 |
| ATM | 108117846 | TGT | T | + | 1058 1059 del GT | C353fs*5 | COSM21924 |
| ATM | 108175462 | G | A | + | 5557 G>A | D1853N | COSM41596 |
| KRAS | 25245350 | c | T | - | 35 G>A | G12D | COSM521 |
| KRAS | 25380275 | A | T | | c.183A>T | Q61H | COSM555 |
| KRAS | 25227343 | C | A | | c.181C>A | Q61K | COSM549 |
| KRAS | 25225628 | G | A | | 436G>A | A146T | COSM19404 |
| PTPNII | 112888210 | G | A | + | 226 G>A | E76K | COSM13000 |
| FLT3 | 28592642 | c | A | - | 2503 G>T | D835V | COSM783 |
| RBI | 48941648 | c | T | + | 958 C>T | R320* | COSM891 |
| PARP2 | 20820412 | A | c | + | 398 A>C | D133A | COSM75849 |
| ARHGAP5 | 32561739 | G | A | + | 1864 G>A | E622K | COSM88502 |
| AKTI | 105246455 | c | T | - | 145 G>A | E49K | COSM36918 |
| AKTI | 104780214 | c | T | - | 49 G>A | E17K | COSM33765 |
| RAD51 | 41001312 | c | T | + | 433 C>T | Q145* | COSM117943 |
| IDH2 | 90631838 | c | T | - | 515 G>A | R172K | COSM33733 |

| Gene | Position | REF | ALT | Strand | CDS | AA | COSID |
|------|----------|-----|-----|--------|-----|-----|-------|
| IDH2 | 90631934 | c | T | - | 419 G>A | R140Q | COSM41590 |
| TP53 | 7577120 | c | T | - | 818 G>A | R273H | COSM10660 |
| TP53 | 7577538 | c | T | - | 743 G>A | R248Q | COSM10662 |
| TP53 | 7577557 | AG | A | - | 723 del C | C242fs*5 | COSM6530 |
| TP53 | 7578406 | c | T | - | 524 G>A | R175H | COSM10648 |
| TP53 | 7579423 | GG | G | - | 263 del C | S90fs*33 | COSM18610 |
| NFI | 29556989 | T | TAC | + | 2987 2988 ins AC | R997fs*16 | COSM41820 |
| NFI | 29576111 | c | T | + | 4084 C>T | R1362* | COSM24443 |
| NFI | 29679317 | TG | T | + | 7501 del G | E2501fs*22 | COSM24468 |
| SMAD4 | 48603093 | T | TT | + | 13941395 ins T | A466fs*28 | COSM14105 |
| AKT2 | 40761084 | c | A | - | 268 G>T | V90L | COSM93894 |
| ERCCI | 45924470 | G | T | - | 287 C>A | A96E | COSM140843 |
| GNAS | 57484420 | c | T | + | 601 C>T | R201C | COSM27887 |
| ERBB2 | 39724728..39724729 | | insGCATACGTGATG | | 2310_2311 ins12 | p.E770_A771 in sAYVM | COSM404915 |

"Diagnose" im Sinne dieser Erfindung bedeutet, dass von der Mutationsrate auf die Tumoraktivität geschlossen werden kann. Je größer die Mutationsrate ist, desto größer ist die Tumoraktivität, insbesondere die Wahrscheinlichkeit einer Metastasierung. Folglich betrifft die Erfindung die Diagnose von Krebs bzw. eines Tumors. Der Begriff der Diagnose umfasst die medizinische Diagnostik und diesbezügliche Untersuchungen, insbesondere die in-vitro Diagnostik und Labordiagnostik. Bevorzugt ist die Aussage über eine Krankheit oder einen Zustand eines Patienten.

Im Rahmen dieser Erfindung wird unter "Patient" ein beliebiger Proband verstanden.

Ein weiterer Gegenstand der Erfindung betrifft einen Kit enthaltend

a.) Teil (z.B. Tube) mit mindestens einer humanen Referenznukleinsäure und einer Nukleinsäure aufweisend eine oder mehrere Mutationen zur Referenzsequenz in einer DNA-freien Serum- oder Plasmaprobe, wobei eine vorbestimmte Allelfrequenz eingestellt ist,
und optional
b.) Teil mit einer humanen Referenznukleinsäure in einer DNA-freien Serum- oder Plasmaprobe,
und optional
c.) Teil mit einer DNA-freien Serum- oder Plasmaprobe,

zur Durchführung eines der vorbeschriebenen Verfahren oder die Verwendung eines solchen Kits zur Durchführung eines der vorbeschriebenen Verfahren.

Nachfolgend wird die Erfindung durch Beispiele erläutert. Die Erfindung ist jedoch nicht auf die Beispiele beschränkt, sondern grundsätzlich universell anwendbar.

Beispiel 1:

Durchführung des erfindungsgemäßen Verfahrens mit Bereitstellung eines Kits:

1) Extraktion von Nukleinsäuren (DNA), insbesondere cfDNA aus dem erfindungsgemäßen Serum- oder Plasma-Referenzmaterial mit kommerziellen Kits, wie z.B. QIAamp ccfDNA/RNA Kit (Qiagen®), PME free-circulating DNA Extraction Kit (AnalytikJena®), MagMAX™ Cell-Free DNA Isolation Kit (ThermoFisher®).

2) Quantifizierung der Nukleinsäuren, insbesondere cfDNA im Eluat fluorimetrisch z.B. mit Qubit® (ThermoFisher®) oder spektrophotometrisch mit NanoDrop® oder ein anderes Spektrometer.

3) Optional kann eine qualitative Analyse der Nukleinsäuren, insbesondere cfDNA vorgenommen werden, wie z.B. eine Fragmentlängenanalyse mit einem Bioanalyzer (Agilent®), Fragmentanalyzer (Agilent®) oder Pulse-Field Gelelektrophorese.

4) Anschließend wird die erhaltene Nukleinsäure, insbesondere cfDNA einer PCR zugeführt.

Parallel kann eine entsprechende Serum- oder Plasmaprobe eines Patienten vorbereitet werden.

Output ddPCR:

Am Beispiel eines QX200 ddPCR Systems von BioRad®:
Mittels ddPCR wird ein definiertes Volumen der zu untersuchenden Nukleinsäure in tausende einzelne Reaktionsräume verteilt. Dabei werden nach der Poisson-Verteilung, die zu untersuchenden DNA-Sequenzen auf diese Reaktionsräume verteilt. In den Reaktionsräumen findet eine Amplifikation der Nukleinsäuren statt, falls die Nukleinsäure vorhanden ist. Die Mutationssequenz löst eine von der Referenzsequenz (Wildtypsequenz) farblich unterscheidbare PCR Reaktion aus.

Mittels einschlägiger Software können die Ergebnisse ausgewertet und in einem Plot, insbesondere 2D-Amplituden Plot dargestellt werden:
Auf der Y-Achse sind die Ereignisse aufgetragen, die im FAM Kanal (blaue Fluoreszenz) detektiert wurden (Channel 1 Amplitude).

Auf der X-Achse sind die Ereignisse aufgetragen, die im HEX/VIC Kanal (grüne Fluoreszenz) detektiert wurden (Channel 2 Amplitude).

Jeder geplottete Punkt steht für einen Reaktionsraum, indem entweder eine Reaktion mit einer Referenzsequenz (Wildtypsequenz) (Q4), einer Mutationssequenz (Q1), mit beiden Sequenzen (Q2) oder mit keiner Sequenz (Q4), weil keine Ziel-Nukleinsäure vorhanden war, stattgefunden hat (Figur Ia), wobei die erhaltenen Punktwolken QI bis Q4 in Figur Ib graphisch dargestellt sind.

Eine Auswertung erfolgt in der Weise, dass die von der Software ermittelten Rohdaten (geplottete Punkte) absolut quantifizierte Zahlen für die Referenzsequenz und Mutationssequenz ergeben.

Tab. 1.: Beispielwerte für Q1 und Q4:

| Well Data | | |
|---|---|---|
| Well | DyeName(s) | Copies/20µLWell |
| C11 | FAM | 477 |
| C11 | VIC | 10632 |

Die Allelfrequenz kann aus diesen Werten ermittelt werden, z.B. händisch oder mittels Software, d.h. die Kopienzahlen für die Referenzsequenz und Mutationssequenz werden nach der folgenden Formel die Allelfrequenz berechnet:

$$(CN_{Mut} * 100\%) / (CN_{Mut} + CN_{Wt}) = VAF \ oder \ MAF$$

$CN_{Mut}$ = Kopienzahl Mutationssequenz

$CN_{Wt}$ = Kopienzahl Referenzsequenz

VAF = Variation Allefrequenz oder MAF = Mutations Allelfreuqenz.

Auf diese Weise durchlaufen die Tubes des Kit (jeweilige Standardprobe mit voreingestellter Allelfrequenz, wie z.B. 0,0, 0,1, 1 und 5% in Figur 2) das erfindungsgemäße Verfahren, wobei vorteilhaft folgende Informationen erhalten werden:

1) Qualitativ: Ist die Mutation in der Mutationssequenz detektierbar, ja/nein?

2) Absolut quantifizierte Kopienzahlen ("CN") für Referenzsequenz und Mutationssequenz,

3) Bestimmung der Allelfrequenz.

Beispiele sind in Figur 2 dargestellt.
Aus den berechneten Werten (Figur 2, Tabelle) kann eine Kalibrierungskurve erstellt werden und die Validierung abgeschlossen werden (Figur 3).
Mittels NGS ist ebenfalls eine leichte Auswertung möglich, da jede Kopie einem "Read" entspricht, und ebenfalls Referenzsequenz und Mutationssequenz leicht unterschieden werden können.
Anhand der Kalibrierungskurve können parallel oder versetzt Patienten- bzw. Probandenproben (supra) bestimmt werden und eine Diagnose durchgeführt werden.

**Patentansprüche**

1. Verfahren zur Validierung eines Polymerase-Chain-Reaction (PCR)-Verfahrens mittels Bestimmung der Allelfrequenz und / oder Mutationsrate in Nukleinsäuren mit den Schritten:

   a.) Bereitstellen mindestens einer humanen Referenznukleinsäure und einer humanen Nukleinsäure aufweisend eine oder mehrere Mutationen zur Referenznukleinsäure in einer DNA-freien Serum- oder Plasmaprobe, wobei eine bestimmte Allelfrequenz und / oder Mutationsrate vorgegeben ist,
   und optional
   b.) Bereitstellen einer humanen Referenznukleinsäure aus a.) in einer DNA-freien Serum- oder Plasmaprobe,
   und optional
   c.) Bereitstellen einer DNA-freien Serum- oder Plasmaprobe,

wobei die bestimmte Allelfrequenz in a.) nachgewiesen wird, und ggfs. mit b.) oder / und c.) verglichen wird.

2. Verfahren zur Validierung eines PCR-Verfahrens mittels Bestimmung der Allelfrequenz und / oder Mutationsrate in Nukleinsäuren nach Anspruch 1, **dadurch gekennzeichnet, dass** das Verfahren für mehrere vorgegebene Allelfrequenzen aus a.) durchgeführt wird.

3. Verfahren zur Validierung eines PCR-Verfahrens mittels Bestimmung der Allelfrequenz und / oder Mutationsrate in Nukleinsäuren nach Anspruch 2, **dadurch gekennzeichnet, dass** eine Validierungs- , Kalibrierungskurve erhalten wird.

4. Verfahren zur Validierung eines PCR-Verfahrens zur Bestimmung der Allelfrequenz und / oder Mutationsrate in Nukleinsäuren nach Anspruch 1, **dadurch gekennzeichnet, dass** die Konzentrationen an Referenznukleinsäure und Mutationsnukleinsäuren in a.) und b.) vorgegeben sind.

5. Verfahren zur Validierung eines PCR-Verfahrens zur Bestimmung der Allelfrequenz und / oder Mutationsrate in Nukleinsäuren nach einem der vorhergehenden Ansprüche, **dadurch gekennzeichnet, dass** die Mutationsnukleinsäure mindestens einen Tumormarker aufweisen.

6. Verfahren zur Validierung eines PCR-Verfahrens zur Bestimmung der Allelfrequenz und / oder Mutationsrate in Nukleinsäuren nach einem der vorhergehenden Ansprüche, **dadurch gekennzeichnet, dass** eine qPCR (real-time quantitative Polymerase Chain Reaction), digital droplet PCR (ddPCR) oder "next generation sequencing" (NGS) durchgeführt wird.

7. Verfahren zur Bestimmung der Allelfrequenz und / oder Mutationsrate mindestens einer Probennukleinsäure mittels eines PCR-Verfahrens, wobei eine Kalibrierung mittels eines Verfahrens nach Anspruch 1 erfolgt ist.

8. Verfahren zur Bestimmung der Allelfrequenz und / oder Mutationsrate mindestens einer Probennukleinsäure mittels eines PCR-Verfahrens, wobei eine quantitative Bestimmung der Probennukleinsäure erfolgt.

9. Verfahren zur Bestimmung der Allelfrequenz und / oder Mutationsrate mindestens einer Probennukleinsäure mittels eines PCR-Verfahrens nach Anspruch 7 oder Anspruch 8, **dadurch gekennzeichnet, dass** die Probennukleinsäure eines Patienten eine cfDNA oder ctDNA ist.

10. Verfahren zur Diagnose oder Prognose einer Tumorerkrankung, wobei eine Änderung der Allelfrequenz und / oder Mutationsrate einer Probennukleinsäure aus einer ersten Probe und einer zweiten und / oder weiteren Probe auf die Früherkennung und Erkennung, zur Beurteilung des Schweregrades und zur therapiebegleitenden Verlaufsbeurteilung schließen lässt, wobei eine Kalibrierung mittels eines Verfahrens nach Anspruch 1 erfolgt ist.

11. Verfahren zur Diagnose oder Prognose einer Tumorerkrankung nach Anspruch 10, wobei die zweite oder weitere Probe zu einem jeweiligen späteren Zeitpunkt von einem Patienten entnommen wird.

12. Kit enthaltend

a.) Teil mit mindestens einer humanen Referenznukleinsäure und einer Nukleinsäure aufweisend eine oder mehrere Mutationen zur Referenzsequenz in einer DNA-freien Serum- oder Plasmaprobe, wobei eine vorbestimmte Allelfrequenz eingestellt ist,
und optional
b.) Teil mit einer humanen Referenznukleinsäure in einer DNA-freien Serum- oder Plasmaprobe,
und optional
c.) Teil mit einer DNA-freien Serum- oder Plasmaprobe,
zur Durchführung eines Verfahrens nach Anspruch 1 oder Anspruch 7 oder Anspruch 10.

13. Verwendung eines Kits nach Anspruch 12 zur Durchführung eines Verfahrens nach Anspruch 1 oder Anspruch 7 oder Anspruch 10.

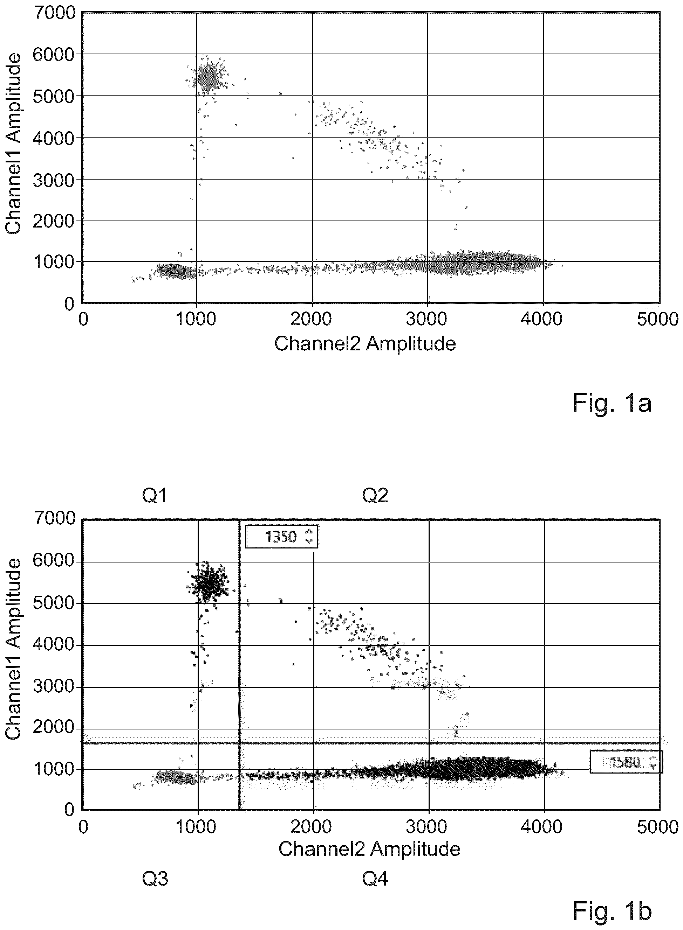

Fig. 1a

Fig. 1b

EGFR-Multiplex
**0%** AF cfDNA
in Plasma

EGFR-Multiplex
**1%** AF cfDNA
in Plasma

| Kanal | CN / 20ml Well | AF [%] |
|---|---|---|
| EGFR-Multiplex 5% AF in Plasma | | |
| FAM EGFR S768I | 0 | 0 |
| VIC EGFR WT | 10.388 | |
| EGFR-Multiplex 0,1% AF in Plasma | | |
| FAM EGFR S768I | 23.86 | 0.09 |
| VIC EGFR WT | 27.516 | |
| EGFR-Multiplex 1% AF in Plasma | | |
| FAM EGFR S768I | 53.6 | 0.7 |
| VIC EGFR WT | 7.938 | |
| EGFR-Multiplex 5% AF in Plasma | | |
| FAM EGFR S768I | 449 | 4.1 |
| VIC EGFR WT | 10.636 | |

Fig. 2/1

| Kanal | CN / 20ml Well | AF[%] |
|---|---|---|
| EGFR-Multiplex 5% AF in Plasma | | |
| FAM EGFR S768I | 0 | 0 |
| VIC EGFR WT | 10.388 | |
| EGFR-Multiplex 0,1% AF in Plasma | | |
| FAM EGFR S768I | 23.86 | 0.09 |
| VIC EGFR WT | 27.516 | |
| EGFR-Multiplex 1% AF in Plasma | | |
| FAM EGFR S768I | 53.6 | 0.7 |
| VIC EGFR WT | 7.938 | |
| EGFR-Multiplex 5% AF in Plasma | | |
| FAM EGFR S768I | 449 | 4.1 |
| VIC EGFR WT | 10.636 | |

Fig. 2/2

Kalibrierungskurve

Fig. 3

**EP 3 851 540 A1**

Europäisches Patentamt
European Patent Office
Office européen des brevets

# EUROPÄISCHER RECHERCHENBERICHT

Nummer der Anmeldung

EP 20 15 2341

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2017/210372 A1 (TRANSLATIONAL GENOMICS RES INST [US]) 7. Dezember 2017 (2017-12-07) * siehe ganzes Dok, bes. Ansprüche und Par. 57-61 * ----- | 1-13 | INV. C12Q1/6806 C12Q1/6886 |
| X | WO 2015/073080 A1 (LIFE TECHNOLOGIES CORP [US]) 21. Mai 2015 (2015-05-21) * sieh ganzes Dok., besonders Ansprüche, Beispiel 1-4, par. 46, 50 * ----- | 1-13 | |

**RECHERCHIERTE SACHGEBIETE (IPC)**

C12Q

Der vorliegende Recherchenbericht wurde für alle Patentansprüche erstellt

| Recherchenort | Abschlußdatum der Recherche | Prüfer |
|---|---|---|
| München | 29. Mai 2020 | Mueller, Frank |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

16

**ANHANG ZUM EUROPÄISCHEN RECHERCHENBERICHT**
**ÜBER DIE EUROPÄISCHE PATENTANMELDUNG NR.**

EP 20 15 2341

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-05-2020

| Im Recherchenbericht angeführtes Patentdokument | Datum der Veröffentlichung | Mitglied(er) der Patentfamilie | Datum der Veröffentlichung |
|---|---|---|---|
| WO 2017210372 A1 | 07-12-2017 | EP 3464593 A1<br>US 2019153509 A1<br>WO 2017210372 A1 | 10-04-2019<br>23-05-2019<br>07-12-2017 |
| WO 2015073080 A1 | 21-05-2015 | EP 3068896 A1<br>US 2015133314 A1<br>US 2019284632 A1<br>WO 2015073080 A1 | 21-09-2016<br>14-05-2015<br>19-09-2019<br>21-05-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018094183 A1